# FASCICULE DE BREVET EUROPEEN

(11) **EP 1 451 197 B1**
(45) Date de publication et mention de la délivrance du brevet: **02.05.2007**
(21) Numéro de dépôt: 02803450.2
(22) Date de dépôt: 20.11.2002
(51) Int. Cl.: C07D 519/00, A61K 31/551, A61P 25/28, A61P 25/16

(54) **DERIVES DE 4-(OXAZOLOPYRIDIN-2-YL)-1,4-DIAZABICYCLO-[3.2.2]-NONANE, LEUR PREPARATION ET LEUR APPLICATION EN THERAPEUTIQUE**
4-(OXAZOLOPYRIDIN-2-YL)-1,4-DIAZABICYCLO-[3.2.2]-NONANDERIVATE, DEREN HERSTELLUNG UND DEREN THERAPEUTISCHE VERWENDUNG
4-(OXAZOLOPYRIDIN-2-YL)-1,4-DIAZABICYCLO-[3.2.2]-NONANE DERIVATIVES, PREPARATION AND THERAPEUTIC USE THEREOF

(30) Priorité: 23.11.2001 FR 0115152
(43) Date de publication de la demande: 01.09.2004
(73) Titulaire: Sanofi-Aventis, 75013 Paris (FR)
(72) Inventeur: GALLI, Frédéric, F-92420 Vaucresson (FR); LECLERC, Odile, F-91300 Massy (FR); LOCHEAD, Alistair, F-94220 Charenton le Pont (FR)
(74) Mandataire: Ludwig, Jacques
(86) Numéro de dépôt international: PCT/FR2002/003978
(87) Numéro de publication internationale: WO 2003/044024

(56) Documents cités:
- EP-A- 1 219 622
- WO-A-00/34279
- US-A- 4 826 848

## Description

La présente invention a pour objet des composés, ligands des récepteurs nicotiniques, utiles dans le traitement ou la prévention des désordres liés à un dysfonctionnement des récepteurs nicotiniques, notamment au niveau du système nerveux central.

Des composés interagissant avec les récepteurs nicotiniques ont été décrits dans l'état de la technique. Par exemple, le document US4,826,848 décrit des dérivés oxazol- et thiazol-amines bicycliques condensés utilisés dans le traitement de la dépression, le traitement de la maladie de Parkinson et le traitement des maladies liées à une perturbation de l'entérokinèse. Le document WO00/34279 concerne des dérivés 1,4-diazabicyclo[3.2.2]nonane présentant une affinité vis-à-vis des récepteurs nicotiniques.

Les composés de la présente invention répondent à la formule générale (I) 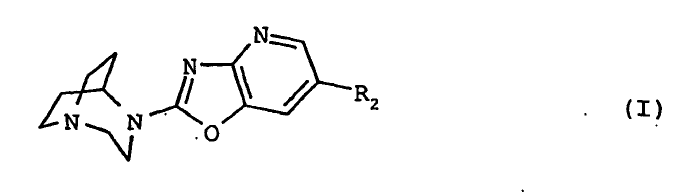 dans laquelle R₂ représente un atome de chlore, un groupe méthyle ou un groupe thién-3-yle.

Les composés de l'invention peuvent exister à l'état de bases ou de sels d'addition à des acides.

Des composés de formule générale (I) sont suggérés dans la demande de brevet EP-1219622 ; toutefois ils n'y sont pas spécifiquement décrits.

Conformément à l'invention, on peut préparer les composés de formule générale (I) par un procédé illustré par le schéma qui suit.

On fait réagir le 1,4-diazabicyclo[3.2.2]nonane de formule (V) avec un composé de formule générale (VI) dans laquelle R₂ est tel que défini ci-dessus, et W représente un atome d'halogène ou un groupe méthylsulfanyle.

Les composés de formule générale (VI) sont accessibles par des méthodes décrites dans la littérature comme par exemple dans *J. Org. Chem.* 1995, **60**(17), 5721.
La préparation du 1,9-diazabicyclo[3.2.2]nonane est décrite dans *J. Med. Chem.* 1993, **36,** 2311-2320.

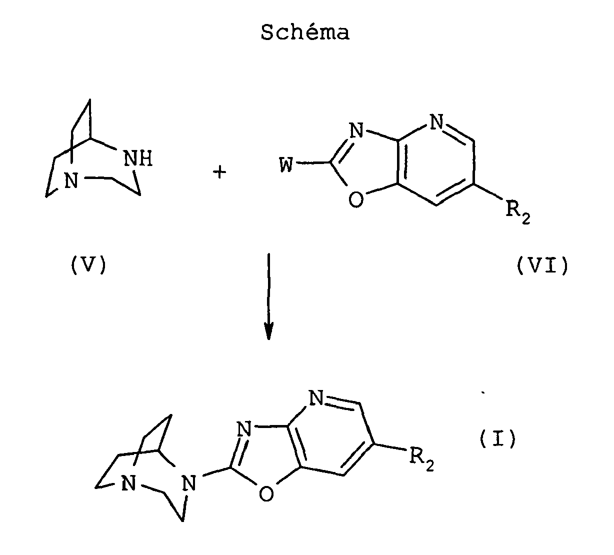

Les exemples qui vont suivre illustrent la préparation de quelques composés de l'invention. Les microanalyses élémentaires, et les spectres I.R. et R.M.N., ainsi que, dans certains cas, les spectres de diffraction aux rayons X, confirment les structures des composés obtenus.
Les numéros indiqués entre parenthèses dans les titres des exemples correspondent à ceux de la 1ère colonne du tableau donné plus loin.
Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

### Exemple 1 (Composé N°1).

### Bromhydrate de 4-(6-thien-3-yl-oxazolo[4,5-b]pyridin-2-yl)-1,4-diazabicyclo[3.2.2]nonane 2:1.

Dans un réacteur de 25 ml on introduit successivement 0,2 g (0,62 mmole) de 4-(6-bromo-oxazolo[4,5-b]pyridin-2-yl)-1,4-diazabicyclo[3.2.2]nonane, 0,079 g (0,62 mmole) d'acide 3-thiophèneboronique, 0,024 g de tétrakis(triphénylphos phino) palladium en suspension dans 10 ml de toluène. On ajoute ensuite 1 ml d'une solution aqueuse 2M de carbonate de sodium et 0,1 ml d'éthanol et on chauffe le mélange au reflux pendant 12 h.
On le verse dans 10 ml d'eau, on extrait la phase aqueuse par du chloroforme, on sèche les phases organiques sur sulfate de magnésium, on les filtre et on les concentre sous pression réduite, et on purifie le résidu par chromatographie sur plaque de silice en éluant par un mélange 90/10/1 de chloroforme, méthanol et ammoniaque. On obtient 0,05 g de produit que l'on dissout dans 10 ml d'acétone pour ajouter 0, 05 ml d'une solution d'acide bromhydrique à 33% dans l'acide acétique. On collecte les cristaux par filtration.
On obtient 0,04 g de produit.
Point de fusion : 316-319°C.

### Exemple 2 (Composé N°2).

### Bromhydrate de 4-(6-méthyl-oxazolo[4,5-b]pyridin-2-yl)-1,4-diazabicyclo[3. 2.2]nonane 2:1.

Dans un réacteur de 10 ml on introduit sous argon successivement 0,3 g (0,93 mmole) de 4-(6-bromo-oxazolo[4,5-b]pyridin-2-yl)-1,4-diazabicyclo[3.2 .2]nonane, 0,004 g (0,02 mmole) de diacétate de palladium, 0,022 g (0,08 mmole) de tri (o-tolyl) phosphine en solution dans 2 ml de diméthylformamide. On ajoute 0,18 ml (1,3 mmole) de triéthylamine et 0,15 ml (1,11 mmole) de tétraméthylétain, et on chauffe le mélange au reflux pendant 4 h.
On dilue le milieu réactionnel avec de l'éther éthylique, on le filtre et on concentre le filtrat sous pression réduite. On purifie le résidu par chromatographie sur colonne de gel de silice en éluant par un mélange 92/8/0,8 de chloroforme, méthanol et ammoniaque. On obtient 0,12 g de produit que l'on dissout dans 20 ml d'acétone pour ajouter 0,24 ml d'une solution d'acide bromhydrique à 33% dans l'acide acétique. On collecte les cristaux par filtration.
On obtient 0,12 g de produit.
Point de fusion : 286-287°C.

Le tableau qui suit illustre les structures chimiques et les propriétés physiques des trois composés de l'invention. Dans la colonne "Sel", "HBr" désigne un bromhydrate. Les rapports molaires acide:base sont indiqués en regard.

**Tableau**

| | | | |
|---|---|---|---|
| 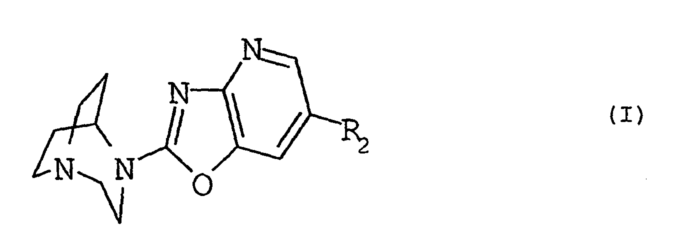 | | | |

| N° | R₂ | Sel | F (°C) |
|---|---|---|---|
| 1 | thién-3-yl | HBr 2:1 | 316-319 |
| 2 | CH₃ | HBr 2:1 | 286-287 |
| 3 | Cl | HBr 2:1 | 298-299 |

Les composés de la présente invention ont été étudiés quant à leur affinité vis à vis des récepteurs nicotiniques contenant la sous-unité α₄β₂ selon les méthodes décrites par Anderson et Arneric dans *Eur. J. Pharmacol*. (1994), **253,** 261 et par Hall et coll. dans *Brain Res*. 1993, **600**, 127.
On décapite des rats mâles Sprague Dawley de 150 à 200 g et on prélève rapidement la totalité du cerveau, on l'homogénéise dans 15 volumes d'une solution de sucrose à 0,32 M à 4°C puis on le centrifuge à 1000 G pendant 10 min. On élimine le culot et centrifuge le surnageant à 20000 G pendant 20 min à 4°C. On récupère le culot et on l'homogénéise à l'aide d'un broyeur Polytron ^{™} dans 15 volumes d'eau bidistillée à 4°C, puis on le centrifuge à 8000 G pendant 20 min. On élimine le culot et on centrifuge le surnageant et la couche de peau (buffy coat) à 40000 G pendant 20 min, on récupère le culot, le remet en suspension dans 15 ml d'eau bidistillée et on le centrifuge encore une fois à 40000 G avant de le conserver à -80°C.

Le jour de l'expérience on décongèle lentement le tissu et on le met en suspension dans 3 volumes de tampon. On fait incuber 150 µl de cette suspension membranaire à 4°C pendant 120 min en présence de 100 µl de [³H]-cytisine à 1 nM dans un volume final de 500 µl de tampon, en présence ou en absence de composé à tester. On arrête la réaction par filtration sur des filtres Whatman GF/B^{™} préalablement traités avec de la polyéthylènimine, on rince les filtres avec deux fois 5 ml de tampon à 4°C, et on mesure la radioactivité retenue sur le filtre par scintigraphie liquide. On détermine la liaison non spécifique en présence de (-)-nicotine à 10 µM ; la liaison non spécifique représente 75 à 85% de la liaison totale récupérée sur le filtre. Pour chaque concentration de composé étudié on détermine le pourcentage d'inhibition de la liaison spécifique de [³H]-cytisine, puis on calcule la CI₅₀, concentration de composé qui inhibe 50% de la liaison spécifique.
Les CI₅₀ des composés de l'invention les plus affins se situent entre 0,6 et 10 µM.

Les composés de l'invention ont aussi été étudiés quant à leur affinité vis-à-vis des récepteurs nicotiniques contenant la sous unité α₇, selon les méthodes décrites par Mark et Collins dans *J*. *Pharmacol. Exp. Ther.* 1982, **22**, 564 et par Marks et coll. dans *Mol. Pharmacol.* 1986, **30,** 427. On décapite des rats mâles OFA de 150 à 200 g, on prélève rapidement la totalité du cerveau, on l'homogénéise à l'aide d'un broyeur Polytron^{™} dans 15 volumes d'une solution de sucrose à 0,32 M à 4°C, puis on le centrifuge à 1000 G pendant 10 min. On élimine le culot et on centrifuge le surnageant à 8000 G pendant 20 min à 4°C. On récupère le culot et on l'homogénéise à l'aide d'un broyeur Polytron^{™} dans 15 volumes d'eau bidistillée à 4°C, puis on le centrifuge à 8000 G pendant 20 min. On élimine le culot et on centrifuge le surnageant et la couche de peau ("buffy coat") à 40000 G pendant 20 min. On récupère le culot, on le remet en suspension avec 15 volumes d'eau bidistillée à 4°C et on le centrifuge encore une fois à 40000 G pendant 20 min avant de le conserver à -80°C.

Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis les membranes sont incubées pendant 60 min à 37°C, à l'obscurité, en présence de 50 µl de [³H]α-bungarotoxine à 1 nM dans un volume final de 250 µl de tampon HEPES 20 mM. On arrête la réaction par filtration sur des filtres Whatman GF/C^{™} préalablement traités pendant 3 heures avec de la polyéthylènimine à 0,05%. On rince les filtres avec deux fois 5 ml de tampon à 4°C et on mesure la radioactivité retenue sur chaque filtre par scintigraphie liquide. On détermine la liaison non spécifique en présence de α-bungarotoxine à 1 µM finale ; la liaison non spécifique représente environ 60% de la liaison totale récupérée sur le filtre. Pour chaque concentration de composé étudié on détermine le pourcentage d'inhibition de la liaison spécifique de [³H]α-bungarotoxine, puis on calcule la CI₅₀, concentration de composé qui inhibe 50% de la liaison spécifique.

Les CI₅₀ des composés de l'invention les plus affins se situent entre 0,001 et 0,6 µM.

Les résultats qui précèdent montrent que les composés de l'invention sont des ligands sélectifs pour les sous unités. α7 du récepteur nicotinique.

Les résultats des essais suggèrent l'utilisation des composés dans le traitement ou la prévention des désordres liés à un dysfonctionnement des récepteurs nicotiniques, notamment au niveau du système nerveux central.

Ces désordres comprennent les altérations cognitives, plus spécifiquement mnésiques, mais également attentionnelles, liées à la maladie d'Alzheimer, au vieillissement pathologique (Age Associated Memory Impairment, AAMI), au syndrome Parkinsonien, à la trisomie 21 (Down's syndrome), au syndrome alcoolique de Korsakoff, aux démences vasculaires (multi-infarct dementia, MDI).
Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.
Les composés de l'invention peuvent également constituer un traitement curatif ou symptomatique des pathologies neurodégénératives aiguës telles que les accidents vasculaires cérébraux et les épisodes hypoxiques cérébraux, ainsi que des pathologies neurodégénératives chroniques telles que la maladie d'Alzheimer. Ils peuvent encore être utilisés dans les cas de pathologies psychiatriques : schizophrénie, dépression, anxiété, attaques de panique, comportements compulsifs et obsessionnels.
Ils peuvent prévenir les symptômes dus au sevrage au tabac, à l'alcool, aux différentes substances induisant une dépendance, telles que cocaïne, LSD, cannabis, benzodiazépines.

C'est pourquoi la présente invention a également pour objet des compositions pharmaceutiques contenant une dose efficace d'au moins un composé selon l'invention, à l'état de base ou de sel ou de solvat pharmaceutiquement acceptable, et en mélange, le cas échéant, avec des excipients convenables.

Lesdits excipients sont choisis selon la forme pharmaceutique et le mode d'administration souhaité.

Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.

Les formes unitaires d'administration peuvent être, par exemple, des comprimés, des gélules, des granules, des poudres, des solutions ou suspensions orales ou injectables, des timbres transdermiques ("patch"), des suppositoires. Pour l'administration topique on peut envisager des pommades, lotions et collyres.
Lesdites formes unitaires sont dosées pour permettre une administration journalière de 0,01 à 20 mg de principe actif par kg de poids corporel, selon la forme galénique.

Pour préparer des comprimés on ajoute au principe actif, micronisé ou non, un véhicule pharmaceutique qui peut être composé de diluants, comme par exemple le lactose, la cellulose microcristalline, l'amidon, et des adjuvants de formulation comme des liants, (polyvinylpyrrolidone, hydroxypropylméthylcellulose, etc), des agents d'écoulement comme la silice, des lubrifiants comme le stéarate de magnésium, l'acide stéarique, le tribehenate de glycerol, le stéarylfumarate de sodium. Des agents mouillants ou tensioactifs tels que le laurylsulfate de sodium peuvent aussi être ajoutés.
Les techniques de réalisation peuvent être la compression directe, la granulation sèche, la granulation humide ou la fusion à chaud.
Les comprimés peuvent être nus, dragéifiés, par exemple par du saccharose, ou enrobés avec divers polymères ou autres matières appropriées. Il peuvent être conçus pour permettre une libération rapide, retardée ou prolongée du principe actif grâce à des matrices polymères ou à des polymères spécifiques utilisés dans l'enrobage.

Pour préparer des gélules on mélange le principe actif avec des véhicules pharmaceutiques secs (simple mélange, granulation sèche ou humide, ou fusion à chaud), liquides ou semi-solides.
Les gélules peuvent être dures ou molles, pelliculées ou non, de manière à avoir une activité rapide, prolongée ou retardée (par exemple pour une forme entérique).

Une composition sous forme de sirop ou d'élixir ou pour l'administration sous forme de gouttes peut contenir le principe actif conjointement à un édulcorant, de préférence acalorique, du méthylparaben ou du propylparaben comme antiseptique, un agent de sapidité et un colorant.

Les poudres et granules dispersibles dans de l'eau peuvent contenir le principe actif en mélange avec des agents de dispersion ou des agents mouillants, ou des agents dispersants comme la polyvinylpyrrolidone, de mêmes qu'avec des édulcorants et des agents correcteurs de goût.

Pour l'administration rectale, on recourt à des suppositoires préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles injectables contenant des agents de dispersion et/ou des mouillants pharmacologiquement compatibles, par exemple le propylèneglycol ou le butylène-glycol.

Le principe actif peut être formulé également sous forme de microcapsules, éventuellement avec un ou plusieurs supports ou additifs, ou bien avec une matrice polymère ou avec une cyclodextrine (timbres transdermiques, formes à libération prolongée).

Les compositions topiques selon l'invention comprennent un milieu compatible avec la peau. Elles peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Enfin, les compositions pharmaceutiques selon l'invention peuvent contenir, à côté d'un composé de formule générale (I), d'autres principes actifs qui peuvent être utiles dans le traitement des troubles et maladies indiqués ci-dessus.

## Revendications

1. Composé répondant à la formule générale (I) 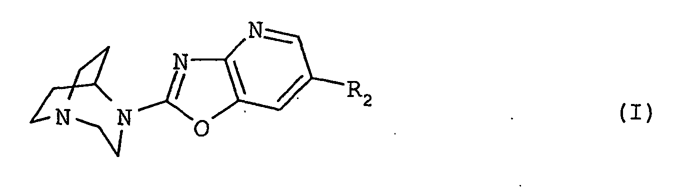 dans laquelle R₂ représente un atome de chlore, un groupe méthyle ou un groupe thién-3-yle,
à l'état de base ou de sel d'addition à un acide.

2. Médicament, **caractérisé en ce qu'**il consiste en un composé selon la revendication 1.

3. Composition pharmaceutique, **caractérisée en ce qu'**elle contient une dose efficace d'au moins un composé selon la revendication 1, à l'état de base ou de sel ou de solvat pharmaceutiquement acceptable, et en mélange, le cas échéant, avec des excipients convenables.

## Claims

1. Compound corresponding to the general formula (I) 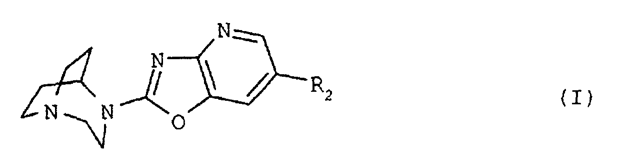 in which R₂ represents a chlorine atom, a methyl group or a 3-thienyl group,
in the form of a base or of an acid-addition salt.

2. Medicinal product, **characterized in that** it consists of a compound according to Claim 1.

3. Pharmaceutical composition, **characterized in that** it contains an effective dose of at least one compound according to Claim 1, in base form or in the form of a pharmaceutically acceptable salt or solvate, and as a mixture, where appropriate, with suitable excipients.

## Patentansprüche

1. Verbindung, die der allgemeinen Formel (I) entspricht: 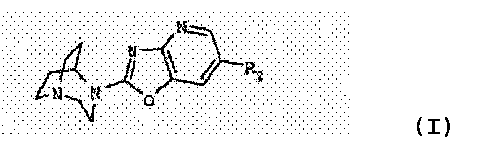 wobei R₂ ein Chloratom, eine Methylgruppe oder eine 3-Thienyl-Gruppe ist,
als Base oder als Säureadditionssalz.

2. Medikament, **dadurch gekennzeichnet, dass** es aus einer Verbindung nach Anspruch 1 besteht.

3. Pharmazeutische Zusammensetzung, **dadurch gekennzeichnet, dass** sie eine wirksame Dosis mindestens einer Verbindung nach Anspruch 1 als Base oder Salz oder Solvat pharmazeutisch annehmbarer Art enthält, gegebenenfalls gemischt mit geeigneten Trägerstoffen.
